# EUROPEAN PATENT SPECIFICATION

(11) **EP 1 942 985 B1**
(45) Date of publication and mention of the grant of the patent: **27.04.2011**
(21) Application number: 06759197.4
(22) Date of filing: 05.05.2006
(51) Int. Cl.: C07K 16/22, A61K 39/395, A61P 9/12, C12N 15/113, A61K 31/713, A61K 31/7088

(54) **METHODS FOR REDUCING BLOOD PRESSURE**
VERFAHREN ZUR SENKUNG DES BLUTDRUCKS
METHODES POUR REDUIRE LA PRESSION SANGUINE

(30) Priority: 29.09.2005 US 722302 P
(43) Date of publication of application: 16.07.2008
(73) Proprietor: FIBROGEN, INC., South San Francisco, CA 94080 (US)
(72) Inventor: LANGSETMO PAROBOK, Ingrid, Montaea, California 94037 (US); SEELEY, Todd, W., Moraga, California 94556 (US); STEPHENSON, Robert, C., Foster City, California 94404 (US)
(74) Representative: Goodfellow, Hugh Robin
(86) International application number: PCT/US2006/017507
(87) International publication number: WO 2007/040636

(56) References cited:
- WO-A-01/66140
- K. HISHIKAWA ET AL: "Static pressure regulates connective tissue growth factor expression in human mesangial cells" THE JOURNAL OF BIOLOGICAL CHEMISTRY, vol. 276, no. 20, 2001, pages 16797-16803, XP002400095
- DATABASE MEDLINE [Online] US NATIONAL LIBRARY OF MEDICINE (NLM), BETHESDA, MD, US; 2007, RANGANATHAN PRATHIBHA ET AL: "Expression profiling of genes regulated by TGF-beta: differential regulation in normal and tumour cells." Database accession no. NLM17425807 & BMC GENOMICS 2007 LNKD- PUBMED:17425807, vol. 8, 2007, page 98, ISSN: 1471-2164
- DATABASE MEDLINE [Online] US NATIONAL LIBRARY OF MEDICINE (NLM), BETHESDA, MD, US; 1996, TAKADA K ET AL: "Endothelin-1 secretion from cultured vascular endothelial cells of DOCA-salt hypertensive rats." Database accession no. NLM8761033 & LIFE SCIENCES 1996 LNKD- PUBMED:8761033, vol. 59, no. 9, 1996, pages PL111-PL116, ISSN: 0024-3205
- DATABASE MEDLINE [Online] US NATIONAL LIBRARY OF MEDICINE (NLM), BETHESDA, MD, US; July 2003 (2003-07), LIJNEN PAUL J ET AL: "Association between transforming growth factor-beta and hypertension." Database accession no. NLM12850397 & AMERICAN JOURNAL OF HYPERTENSION JUL 2003 LNKD- PUBMED:12850397, vol. 16, no. 7, July 2003 (2003-07), pages 604-611, ISSN: 0895-7061

## Description

### FIELD OF THE INVENTION

The present invention relates to methods and agents for reducing blood pressure. Methods and agents for reducing diastolic blood pressure, for reducing systolic blood pressure, and for reducing mean arterial pressure are also provided.

### BACKGROUND OF THE INVENTION

Elevated blood pressure (e.g., any blood pressure at levels above normal levels) and hypertension are significant public health problems. Numerous risk factors are associated with elevations in blood pressure or the development of hypertension, including age, race, family history, obesity, inactivity, tobacco use, alcohol use, diet, diabetes, and stress.

Individuals having elevated blood pressure or hypertension are at a significantly greater risk for developing numerous disorders and complications. The extent and severity of these disorders and complications suggest an urgent need for early and effective treatment strategies that reduce blood pressure and that treat hypertension or prevent/reverse the progression of hypertension. Relatively minor reductions in blood pressure can significantly reduce the co-morbidities and co-mortalities associated with hypertension. For example, in adults aged 40-69, a 20 mm Hg reduction in systolic blood pressure (approximately equivalent to a 10 mm Hg reduction in diastolic blood pressure) was associated with a greater than two-fold reduction in death due to stroke and other vascular diseases. (Lewington et al (2002) Lancet 360:1903-1913.)

Therefore, there is a need in the art for methods for reducing blood pressure, treating hypertension, and for methods for treating disorders and complications associated with elevated blood pressure or hypertension or for preventing the development of such disorders and complications. WO-A-01/66140 discloses the use of TGF-beta antagonists to treat or prevent loss of renal function, as well as prevent systemic hypertension. Hishikawa et al (J. Biol Chem. 2001 May 18; 276 (20):16797-803) discloses that high blood pressure up-regulates CTGF expression in mesangial cells.

The present invention meets this need by providing novel methods and agents for use in lowering blood pressure. The present inventors show that specific inhibition of connective tissue growth factor (CTGF) in a subject, e.g., through administration of an anti-CTGF agent, reduces blood pressure in the subject. Methods and agents for reducing blood pressure, and for treating hypertension, and methods for treating disorders and complications associated with elevated blood pressure or hypertension or for preventing the development of such disorders and complications are also provided.

### BRIEF DESCRIPTION OF THE DRAWINGS

Figure 1 sets forth data showing that methods and agents of the present invention effectively reduced systolic blood pressure in mammalian subjects.
Figure 2 sets forth data showing that methods and agents of the present invention effectively reduced diastolic blood pressure in mammalian subjects.
Figure 3 sets forth data showing that methods and agents of the present invention effectively reduced mean arterial pressure in mammalian subjects.
Figure 4 sets forth data showing that methods and agents of the present invention effectively reduced angiotensin II type I receptor gene expression in mammalian subjects.

### SUMMARY OF THE INVENTION

The present invention relates in part to the discovery that connective tissue growth factor (CTGF) plays a key role in systolic and diastolic blood pressure.

The present invention relates to methods and agents for use in reducing blood pressure. In particular, the present invention demonstrates that administration of an anti-CTGF agent effectively reduced both systolic and diastolic blood pressure in human subjects and in other mammalian subjects.

Methods and agents for reducing blood pressure and for treating hypertension, and methods for treating disorders and complications associated with elevated blood pressure or hypertension or for preventing the development of such disorders and complications are also disclosed.

In one embodiment, the invention provides an agent for use in reducing blood pressure in a subject having elevated blood pressure, high blood pressure or hypertension. The invention also provides the use of claim 14. In preferred embodiments of the present invention, the subject is a mammalian subject. Most preferably, the subject is a human subject.

The anti-CTGF agent inhibits CTGF and is an antibody that binds to CTGF, an antisense molecule that inhibits CTGF expression or an SIRNA that inhibits CTGF expression.

In certain embodiments where the subject is a human subject, the subject is selected from the group consisting of a subject having high normal blood pressure, a subject having high blood pressure, and a subject having hypertension. In specific embodiments of the present invention, the hypertension is further selected from group consisting of mild hypertension, moderate hypertension, severe hypertension, and very severe hypertension.

In other embodiments where the subject is a human subject, the subject is a subject having or at risk for having diabetes. The diabetes can be selected, in various embodiments, from the group consisting of type 1 diabetes and type 2 diabetes.

It is contemplated in certain aspects of the present invention that the subject is a human subject at risk for developing elevated blood pressure or hypertension. Such subjects can include a subject having one or more of various risk factors known to be associated with an increased risk of developing elevated or high blood pressure or hypertension. Such risk factors include, for example, family history of high blood pressure, diabetes, obesity, certain ethnic or racial heritage, a sedentary lifestyle, age, alcohol use, tobacco use, caffeine use, diet, sodium sensitivity and salt intake, kidney disease and renal insufficiency, sleep apnea, pregnancy, cirrhosis, Cushing's disease, certain medications, emotional factors, stress, etc.

In certain aspects, the subject at risk is a subject previously treated with or currently taking a blood pressure medication or one or more blood pressure medications including angiotensin converting enzyme inhibitors (e.g., benazepril, fosinopril, lisinopril, quinapril), angiotensin II receptor blockers (e.g., losartan), beta-blockers (e.g., metoprolol tartrate, betaxolol,valsartan), diuretics (e.g., hydrochlorothiazide), vasodilators (e.g., isosorbide dinitrate), alpha-blockers, calcium channel blockers, and statins. It is specifically contemplated herein that, in particular aspects, the subject at risk can be a subject that does not have elevated blood pressure or a subject having normal or even lower than normal blood pressure, e.g., systolic blood pressure at or below 120 mm Hg or diastolic blood pressure at or below 80 mm Hg.

A method for treating or preventing hypertension in a subject, the method comprising administering to the subject an effective amount of an anti-CTGF agent, thereby treating or preventing hypertension in the subject, is also described herein.

The invention further encompasses agents useful in methods for treating or preventing a disorder associated with elevated blood pressure or hypertension in a subject, the methods comprising administering to the subject an effective amount of an anti-CTGF agent, thereby treating the disorder in the subject. In various aspects, the disorder is selected from the group consisting of cardiovascular disease, myocardial infarction, congestive heart failure, cardiac hypertrophy, coronary artery disease, cardiac arrhythmias, atherosclerosis, arteriosclerosis, nephropathy, retinopathy, neuropathy, stroke, obesity, diabetes, bone disease, sexual dysfunction, and metabolic syndrome.

In various embodiments of the present invention, the subject in need is a subject having kidney disease or a nephropathy, in particular, diabetic nephropathy.

It is specifically contemplated that, in various embodiments of each of the methods described herein, the preferred subject is a human subject.

An anti-CTGF agent, as the term is used herein, is an agent that inhibits the expression or activity of CTGF, selected from an antibody that binds to CTGF, an antisense molecule that inhibits CTGF expression, or siRNAs that inhibit CTGF expression. Use of a human monoclonal antibody directed against CTGF is specifically contemplated. In a particular embodiment, the antibody is CLN-1, as described in International Publication No. WO 2004/108764.

The present disclosure contemplates the use of the present methods in combination with other therapies. See discussion, infra. In one embodiment, the method is used in combination with another therapeutic approach to treat elevated or high blood pressure, hypertension or associated disorders and complications, such as, for example, angiotensin converting enzyme inhibitors, angiotensin II receptor blockers, beta-blockers, alpha-blockers, calcium channel blockers, diuretics, vasodilators, etc.

The invention further encompasses agents for use in a method for modulating expression of a gene associated with increased blood pressure in a subject, the method comprising administering to the subject an effective amount of an anti-CTGF agent, thereby modulating expression of the gene in the subject. In certain embodiments, the methods of the present invention reduce expression of a gene associated with increased blood pressure. In one embodiment, the gene encodes angiotensin II type I receptor, and methods are provided for reducing expression of this gene.

### DESCRIPTION OF THE INVENTION

It must be noted that as used herein and in the appended claims, the singular forms "a," "an," and "the" include plural references unless context clearly dictates otherwise. Thus, for example, a reference to "a fragment" includes a plurality of such fragments, a reference to an "antibody" is a reference to one or more antibodies and to equivalents thereof known to those skilled in the art, and so forth.

Unless defined otherwise, all technical and scientific terms used herein have the same meanings as commonly understood by one of ordinary skill in the art to which this invention belongs. Although any methods and materials similar or equivalent to those described herein can be used in the practice or testing of the present invention, the preferred methods, devices, and materials are now described. All publications cited herein are cited for the purpose of describing and disclosing the methodologies, reagents, and tools reported in the publications, which might be used in connection with the invention. Nothing herein is to be construed as an admission that the invention is not entitled to antedate such disclosure by virtue of prior invention.

The practice of the present invention will employ, unless otherwise indicated, conventional methods of chemistry, biochemistry, molecular biology, cell biology, genetics, immunology and pharmacology, within the skill of the art. Such techniques are explained fully in the literature. See, e.g., Gennaro, A.R., ed. (1990) Remington's Pharmaceutical Sciences, 18th ed., Mack Publishing Co.; Colowick, S. et al., eds., Methods In Enzymology, Academic Press, Inc.; Handbook of Experimental Immunology, Vols. I-IV (D.M. Weir and C.C. Blackwell, eds., 1986, Blackwell Scientific Publications); Maniatis, T. et al., eds. (1989) Molecular Cloning: A Laboratory Manual, 2nd edition, Vols. I-III, Cold Spring Harbor Laboratory Press; Ausubel, F. M. et al., eds. (1999) Short Protocols in Molecular Biology, 4th edition, John Wiley & Sons; Ream et al., eds. (1998) Molecular Biology Techniques: An Intensive Laboratory Course, Academic Press); PCR (Introduction to Biotechniques Series), 2nd ed. (Newton & Graham eds., 1997, Springer Verlag).

The present invention relates in part to the discovery that connective tissue growth factor (CTGF) plays a key role in systolic and diastolic blood pressure.

The present invention relates to agents for use in reducing blood pressure. In particular, the present invention demonstrates that administration of an anti-CTGF agent effectively reduced both systolic and diastolic blood pressure in human subjects.

Agents for reducing blood pressure, treating hypertension, and methods for treating disorders and complications associated with elevated blood pressure or hypertension or for preventing the development of such disorders and complications are also provided.

The invention provides agents for reducing blood pressure, treating hypertension, etc.; The agents are used to reduce blood pressure in subjects having elevated or high blood pressure or hypertension. The determination as to whether the subject is such a subject can be made by any measure accepted and utilized by those skilled in the art. For example, a human subject having a systolic blood pressure of below 120 mm Hg and a diastolic blood pressure of below 80 mm Hg may be considered a subject having optimal blood pressure. A human subject having a systolic blood pressure of between 120 to 130 mm Hg or a diastolic blood pressure of between 80 to 85 mm Hg may be considered a subject having normal blood pressure. A human subject having a systolic blood pressure of between 130 to 139 mm Hg or a diastolic blood pressure of between 85 to 89 mm Hg may be considered a subject having high normal blood pressure.

A human subject having a systolic blood pressure of greater than about 140 mm Hg or a diastolic blood pressure of greater than about 90 mm Hg may be considered a subject having high blood pressure or having hypertension. Additionally, a human subject may be considered as having mild hypertension (Stage 1, systolic blood pressure of between 140 to 159 mm Hg; diastolic blood pressure of between 90 to 99 mm Hg), moderate hypertension (Stage 2, systolic blood pressure of between 160 to 179 mm Hg; diastolic blood pressure of between 100 to 109 mm Hg), severe hypertension (Stage 3, systolic blood pressure of between 180 to 209 mm Hg; diastolic blood pressure of between 110 to 119 mm Hg), or very severe hypertension (Stage 4, systolic blood pressure of greater than 210 mm Hg; diastolic blood pressure of greater than 120 mm Hg).

Therefore, in certain embodiments, a preferred human subject suitable for treatment using the present methods and agents is a subject having high normal blood pressure, mild hypertension, moderate hypertension, severe hypertension, or very severe hypertension.

In other embodiments, a preferred human subject is a subject at risk for developing mild hypertension, moderate hypertension, severe hypertension, or very severe hypertension. A subject at risk can be identified, for example, by an assessment of one or more various factors known to be associated with an increased risk of developing high blood pressure or hypertension. Such risk factors include, for example, family history of high blood pressure, diabetes, obesity, certain ethnic or racial heritage, a sedentary lifestyle, age, alcohol use, tobacco use, caffeine use, diet, sodium sensitivity and salt intake, kidney disease and renal insufficiency, sleep apnea, pregnancy, cirrhosis, Cushing's disease, certain medications, emotional factors, stress, etc. In certain aspects, the subject at risk is a subject previously treated with or currently taking a blood pressure medication or one or more blood pressure medications including angiotensin converting enzyme inhibitors (e.g., benazepril, fosinopril, lisinopril, quinapril), angiotensin II receptor blockers (e.g., losartan), beta-blockers (e.g., metoprolol tartrate, betaxolol,valsartan), diuretics (e.g., hydrochlorothiazide), vasodilators (e.g., isosorbide dinitrate), alpha-blockers, calcium channel blockers, and statins. It is specifically contemplated herein that, in particular aspects, the subject at risk can be a subject without elevated blood pressure, e.g., a subject having normal or even lower than normal blood pressure, e.g., systolic blood pressure at or below 120 mm Hg or diastolic blood pressure at or below 80 mm Hg.

Elevated blood pressure and hypertension are common complications of diabetes. Therefore, in certain embodiments of the present invention, it is specifically contemplated that the subject in need is a subject having or at risk for having diabetes or having or at risk for having diabetic complications. In specific embodiments, the diabetes is type 1 diabetes or type 2 diabetes. Elevated blood pressure and hypertension are frequently associated with kidney disease and various nephropathies, including diabetic nephropathy. Thus, in various embodiments of the present invention, the subject in need is a subject having kidney disease or a nephropathy, in particular, diabetic nephropathy.

The present invention provides agents for reducing blood pressure or treating or preventing hypertension in a subject in need, the method comprising administering to the subject an effective amount of an anti-CTGF agent, thereby reducing blood pressure or treating or preventing hypertension in the subject. In certain embodiments, methods and agents of the present invention reduce systolic blood pressure. In other embodiments, methods and agents of the present invention reduce diastolic blood pressure. In a preferred embodiment, both systolic blood pressure and diastolic blood pressure are reduced by methods and agents of the present invention.

Methods for reducing mean arterial pressure (MAP) and pulse pressure are also encompassed by the present invention. Mean arterial pressure can be determined according to the following formula: MAP = [2/3 (systolic blood pressure minus diastolic blood pressure) plus diastolic blood pressure]. Pulse pressure can be determined according to the following formula: pulse pressure = (systolic blood pressure minus diastolic blood pressure). In one aspect, the invention provides a method for reducing mean arterial pressure in a subject in need, the method comprising administering to the subject an effective amount of an anti-CTGF agent, thereby reducing mean arterial pressure in the subject. In another aspect, methods are also provided for reducing pulse pressure in a subject in need, the method comprising administering to the subject an effective amount of an anti-CTGF agent, thereby reducing pulse pressure in the subject.

Essential hypertension, also known as primary or idiopathic hypertension, accounts for approximately 90% of all hypertension cases. The causes of essential hypertension are unknown, but may be associated with various complications and abnormalities in major organs and body systems, including the heart, kidneys, blood vessels, nerves, and hormones. In secondary hypertension, accounting for approximately 5% of hypertension cases, hypertension results from another underlying condition, such as, for example, kidney disease (renal hypertension), adrenal disease (endocrine or adrenal hypertension), thyroid disease, abnormal blood vessels, preeclampsia, sleep apnea, acromegaly, hypercalcemia, oral contraceptives, etc. Isolated systolic hypertension, which is associated with ateriosclerosis, occurs when systolic blood pressure is over 160 mm Hg but diastolic blood pressure is normal. Pregnancy induced hypertension occurs during pregnancy if blood pressure increases by more than 15 mm Hg above normal levels. The present invention encompasses methods and agents for treating or reducing essential hypertension, secondary hypertension, isolated hypertension, and pregnancy induced hypertension.

In various aspects, the present invention provides agents for treating or preventing a disorder associated with elevated blood pressure or hypertension in a subject having or at risk for developing elevated blood pressure or hypertension, comprising administering to the subject an anti-CTGF agent, thereby treating the disorder. Methods for reducing the progression or severity of a disorder associated with elevated blood pressure or hypertension in a subject having or at risk for developing elevated blood pressure or hypertension, the methods comprising administering to the subject an effective amount of an anti-CTGF agent, thereby reducing the progression or severity of the disorder, are also described.

In certain embodiments, the disorder associated with elevated blood pressure or hypertension is a stroke or the risk of having a stroke. In other embodiments, the disorder can be cardiovascular disease, including heart disease, such as, for example, myocardial infarction, congestive heart failure, cardiac hypertrophy, coronary artery disease, cardiac arrhythmias, atherosclerosis, arteriosclerosis, etc. In other embodiments, the disorder is kidney disease, including diabetic nephropathy; retinopathy; loss of bone mineral density; sexual dysfunction; metabolic syndrome, etc.

In one aspect, the invention contemplates agents for reducing or preventing damage to or dysfunction of blood vessels in a subject having elevated or high blood pressure or hypertension or at risk for developing elevated or high blood pressure or hypertension, the method comprising administering to a subject an effective amount of an anti-CTGF agent, thereby reducing or preventing damage to or dysfunction of blood vessels in the subject. In various aspects, the blood vessels can be blood vessels of the macrovasculature, e.g., major blood vessels such as the aorta, the coronary arteries, the carotid arteries, the cerebrovascular arteries, the renal arteries, the iliac arteries, the femoral arteries, the popliteal arteries, or can be blood vessels of the microvasculature, e.g., small blood vessels such as the retinal arterioles, the glomerular arterioles, the vasa nervorum, the cardiac arterioles, and associated capillary beds of the eye, the kidney, the heart, and the central and peripheral nervous system.

In another aspect, the invention contemplates agents for reducing or preventing damage to or dysfunction of tissues and organs in a subject having elevated or high blood pressure or hypertension or at risk for developing elevated or high blood pressure or hypertension, comprising administering to the subject an effective amount of an anti-CTGF agent, thereby reducing or preventing damage to or dysfunction of tissues and organs in the subject. In various aspects, the tissues and organs can include the heart, kidneys, eyes, blood vessels, brain, central nervous system, peripheral nervous system, bones, etc.

In preferred embodiments described herein, the subject is a human subject.

Angiotensin II regulates many aspects of the cardiovascular system, and the angiotensin II type 1 receptor is associated with the regulation of various cardiovascular effects of angiotensin II. (Murphy et al (1991) Nature 351:233-236.) The angiotensin II type 1 receptor is mainly expressed in smooth muscle cells of the vasculature and is associated with modulation of vascular resistance and blood pressure; accordingly, the angiotensin II type 1 receptor is the target for many current anti-hypertensive therapies currently in use. Blockade of angiotensin II type 1 receptors provides clinical benefit in reducing mortality and morbidity endpoints in patients with left ventricular dysfunction following myocardial infarction and congestive heart failure, particularly in type 2 diabetic subjects with renal dysfunction and in high-risk hypertensive patients. (See, e.g., de la Sierra (2006) Cardiovasc Hematol Agents Med Chem 4:67-73.) Therefore, reduction in the expression of the angiotensin II type 1 receptor may lower morbidity and mortality in patients with cardiovascular complications of diabetes.

The invention further encompasses an agent for modulating expression of a gene associated with increased blood pressure in a subject, comprising administering to the subject an effective amount of an anti-CTGF agent, thereby modulating expression of the gene in the subject. In certain embodiments, the methods of the present invention reduce expression of a gene associated with increased blood pressure. In one embodiment, the gene encodes angiotensin II type I receptor, and methods are provided for reducing expression of this gene.

### Anti-CTGF agents

The anti-CTGF agent is an antibody that binds to CTGF, an antisense molecule that inhibits CTGF expression, or siRNAs, that inhibit CTGF expression. Use of anti-CTGF agent, for example, a human monoclonal antibody directed against CTGF, is specifically contemplated.

Exemplary antibodies for use in the methods of the present invention are described, e.g., in U.S. Patent No. 5,408,040; International Publication No. WO 99/07407; International Publication No. WO 99/33878; and International Publication No. WO 00/35936. An exemplary antibody for use in the methods of the present invention is described in International Publication No. WO 2004/108764. Such antibodies, or fragments thereof, can be administered by various means known to those skilled in the art. For example, antibodies are often injected intravenously, intraperitoneally, or subcutaneously.

Small interfering ribonucleic acids (siRNAs), and anti-sense sequences may be used in the present methods to inhibit expression and/or production of CTGF. (See, e.g., Kondo et al. (2000) Biochem Biophys Res Commun 278:119-124.) Such techniques are well-known to those of skill in the relevant art. Anti-sense constructs that target CTGF expression have been described and utilized to reduce CTGF expression in various cell types. (See, e.g., International Publication No. WO 96/38172; International Publication No. WO 00/27868; International Publication No. WO 00/35936; International Publication No. WO 03/053340; Kothapalli et al. (1997) Cell Growth Differ 8(1):61-68; Shimo et al. (1998) J Biochem (Tokyo) 124(1):130-140; and Uchio et al. (2004) Wound Repair Regen 12:60-66.) Such antisense constructs can be used to reduce expression of CTGF and thereby ameliorate or prevent the pathological processes associated with CTGF, such as, for example, elevated blood pressure or hypertension. Such constructs can be designed using appropriate vectors and expressional regulators for cell- or tissue-specific expression and constitutive or inducible expression. Such genetic constructs can be formulated and administered according to established procedures within the art.

Accordingly, in certain embodiments of the present invention, the anti-CTGF agent is an antibody to CTGF. In a particular embodiment, the antibody is a monoclonal antibody to CTGF. In a specific embodiment, the antibody is a human or humanized antibody to CTGF. In a particular embodiment, the antibody is CLN-1, as described in International Publication No. WO 2004/108764. In particular embodiments, the agent is an antisense oligonucleotide or an siRNA.

The present invention contemplates the use of the present agents in combination with other therapies. In one embodiment, the agent is used in combination with another therapy, e.g., to further augment therapeutic effect on certain pathological events, etc. The two treatments may be administered at the same time or consecutively, e.g., during a treatment time course or following disease progression and remission. In another embodiment, the agent is used in combination with another therapeutic method having a similar or different mode of action, e.g., an angiotensin-converting enzyme inhibitor, angiotensin II receptor blockers, statin, etc. Current therapeutic approaches to treat elevated or high blood pressure and hypertension, and associated disorders and complications, are known by one of skill in the art, and include, for example, angiotensin converting enzyme inhibitors, angiotensin II receptor blockers, beta-blockers, alpha-blockers, calcium channel blockers, diuretics, vasodilators, etc. Use of any of these therapeutic agents in combination with the use of compounds of the present invention is specifically contemplated.

### Pharmaceutical Formulations And Routes Of Administration

The compositions of the present invention can be delivered directly or in pharmaceutical compositions containing excipients, as is well known in the art. Present methods of treatment can comprise administration of an effective amount of a compound of the present invention to a subject having or at risk for having high blood pressure or hypertension. In a preferred embodiment, the subject is a mammalian subject, and in a most preferred embodiment, the subject is a human subject.

An effective amount, e.g., dose, of compound or drug can readily be determined by routine experimentation, as can an effective and convenient route of administration and an appropriate formulation. Various formulations and drug delivery systems are available in the art. (See, e.g., Gennaro, ed. (2000) Remington's Pharmaceutical Sciences, supra; and Hardman, Limbird, and Gilman, eds. (2001) The Pharmacological Basis of Therapeutics, *supra*.)

Suitable routes of administration may, for example, include oral, rectal, topical, nasal, pulmonary, ocular, intestinal, and parenteral administration. Primary routes for parenteral administration include intravenous, intramuscular, and subcutaneous administration. Secondary routes of administration include intraperitoneal, intra-arterial, intra-articular, intracardiac, intracisternal, intradermal, intralesional, intraocular, intrapleural, intrathecal, intrauterine, and intraventricular administration. The indication to be treated, along with the physical, chemical, and biological properties of the drug, dictate the type of formulation and the route of administration to be used, as well as whether local or systemic delivery would be preferred.

Pharmaceutical dosage forms of a compound of the invention may be provided in an instant release, controlled release, sustained release, or target drug-delivery system. Commonly used dosage forms include, for example, solutions and suspensions, (micro-) emulsions, ointments, gels and patches, liposomes, tablets, dragees, soft or hard shell capsules, suppositories, ovules, implants, amorphous or crystalline powders, aerosols, and lyophilized formulations. Depending on route of administration used, special devices may be required for application or administration of the drug, such as, for example, syringes and needles, inhalers, pumps, injection pens, applicators, or special flasks. Pharmaceutical dosage forms are often composed of the drug, an excipient(s), and a container/closure system. One or multiple excipients, also referred to as inactive ingredients, can be added to a compound of the invention to improve or facilitate manufacturing, stability, administration, and safety of the drug, and can provide a means to achieve a desired drug release profile. Therefore, the type of excipient(s) to be added to the drug can depend on various factors, such as, for example, the physical and chemical properties of the drug, the route of administration, and the manufacturing procedure. Pharmaceutically acceptable excipients are available in the art, and include those listed in various pharmacopoeias. (See, e.g., USP, JP, EP, and BP, FDA web page (www.fda.gov), Inactive Ingredient Guide 1996, and Handbook of Pharmaceutical Additives, ed. Ash; Synapse Information Resources, Inc. 2002.)

Pharmaceutical dosage forms of a compound of the present invention may be manufactured by any of the methods well-known in the art, such as, for example, by conventional mixing, sieving, dissolving, melting, granulating, dragee-making, tabletting, suspending, extruding, spray-drying, levigating, emulsifying, (nano/micro-) encapsulating, entrapping, or lyophilization processes. As noted above, the compositions of the present invention can include one or more physiologically acceptable inactive ingredients that facilitate processing of active molecules into preparations for pharmaceutical use.

Proper formulation is dependent upon the desired route of administration. For intravenous injection, for example, the composition may be formulated in aqueous solution, if necessary using physiologically compatible buffers, including, for example, phosphate, histidine, or citrate for adjustment of the formulation pH, and a tonicity agent, such as, for example, sodium chloride or dextrose. For transmucosal or nasal administration, semisolid, liquid formulations, or patches may be preferred, possibly containing penetration enhancers. Such penetrants are generally known in the art. For oral administration, the compounds can be formulated in liquid or solid dosage forms and as instant or controlled/sustained release formulations. Suitable dosage forms for oral ingestion by a subject include tablets, pills, dragees, hard and soft shell capsules, liquids, gels, syrups, slurries, suspensions, and emulsions. The compounds may also be formulated in rectal compositions, such as suppositories or retention enemas, e.g., containing conventional suppository bases such as cocoa butter or other glycerides.

Solid oral dosage forms can be obtained using excipients, which may include, fillers, disintegrants, binders (dry and wet), dissolution retardants, lubricants, glidants, antiadherants, cationic exchange resins, wetting agents, antioxidants, preservatives, coloring, and flavoring agents. These excipients can be of synthetic or natural source. Examples of such excipients include cellulose derivatives, citric acid, dicalcium phosphate, gelatine, magnesium carbonate, magnesium/sodium lauryl sulfate, mannitol, polyethylene glycol, polyvinyl pyrrolidone, silicates, silicium dioxide, sodium benzoate, sorbitol, starches, stearic acid or a salt thereof, sugars (i.e. dextrose, sucrose, lactose, etc.), talc, tragacanth mucilage, vegetable oils (hydrogenated), and waxes. Ethanol and water may serve as granulation aides. In certain instances, coating of tablets with, for example, a taste-masking film, a stomach acid resistant film, or a release-retarding film is desirable. Natural and synthetic polymers, in combination with colorants, sugars, and organic solvents or water, are often used to coat tablets, resulting in dragees. When a capsule is preferred over a tablet, the drug powder, suspension, or solution thereof can be delivered in a compatible hard or soft shell capsule.

In one embodiment, the compounds of the present invention can be administered topically, such as through a skin patch, a semi-solid or a liquid formulation, for example a gel, a (micro-) emulsion, an ointment, a solution, a (nano/micro)-suspension, or a foam. The penetration of the drug into the skin and underlying tissues can be regulated, for example, using penetration enhancers; the appropriate choice and combination of lipophilic, hydrophilic, and amphiphilic excipients, including water, organic solvents, waxes, oils, synthetic and natural polymers, surfactants, emulsifiers; by pH adjustment; and use of complexing agents. Other techniques, such as iontophoresis, may be used to regulate skin penetration of a compound of the invention. Transdermal or topical administration would be preferred, for example, in situations in which local delivery with minimal systemic exposure is desired.

For administration by inhalation, or administration to the nose, the compounds for use according to the present invention are conveniently delivered in the form of a solution, suspension, emulsion, or semisolid aerosol from pressurized packs, or a nebuliser, usually with the use of a propellant, e.g., halogenated carbons dervided from methan and ethan, carbon dioxide, or any other suitable gas. For topical aerosols, hydrocarbons like butane, isobutene, and pentane are useful. In the case of a pressurized aerosol, the appropriate dosage unit may be determined by providing a valve to deliver a metered amount. Capsules and cartridges of, for example, gelatin, for use in an inhaler or insufflator, may be formulated. These typically contain a powder mix of the compound and a suitable powder base such as lactose or starch.

Compositions formulated for parenteral administration by injection are usually sterile and, can be presented in unit dosage forms, e.g., in ampoules, syringes, injection pens, or in multi-dose containers, the latter usually containing a preservative. The compositions may take such forms as suspensions, solutions, or emulsions in oily or aqueous vehicles, and may contain formulatory agents, such as buffers, tonicity agents, viscosity enhancing agents, surfactants, suspending and dispersing agents, antioxidants, biocompatible polymers, chelating agents, and preservatives. Depending on the injection site, the vehicle may contain water, a synthetic or vegetable oil, and/or organic co-solvents. In certain instances, such as with a lyophilized product or a concentrate, the parenteral formulation would be reconstituted or diluted prior to administration. Depot formulations, providing controlled or sustained release of a compound of the invention, may include injectable suspensions of nano/micro particles or nano/micro or non-micronized crystals. Polymers such as poly(lactic acid), poly(glycolic acid), or copolymers thereof, can serve as controlled/sustained release matrices, in addition to others well known in the art. Other depot delivery systems may be presented in form of implants and pumps requiring incision.

Suitable carriers for intravenous injection for the molecules of the invention are well-known in the art and include water-based solutions containing a base, such as, for example, sodium hydroxide, to form an ionized compound, sucrose or sodium chloride as a tonicity agent, for example, the buffer contains phosphate or histidine. Co-solvents, such as, for example, polyethylene glycols, may be added. These water-based systems are effective at dissolving compounds of the invention and produce low toxicity upon systemic administration. The proportions of the components of a solution system may be varied considerably, without destroying solubility and toxicity characteristics. Furthermore, the identity of the components may be varied. For example, low-toxicity surfactants, such as polysorbates or poloxamers, may be used, as can polyethylene glycol or other co-solvents, biocompatible polymers such as polyvinyl pyrrolidone may be added, and other sugars and polyols may substitute for dextrose.

For composition useful for the present methods of treatment, a therapeutically effective dose can be estimated initially using a variety of techniques well-known in the art. Initial doses used in animal studies may be based on effective concentrations established in cell culture assays. Dosage ranges appropriate for human subjects can be determined, for example, using data obtained from animal studies and cell culture assays.

A therapeutically effective dose or amount of a compound, agent, or drug of the present invention refers to an amount or dose of the compound, agent, or drug that results in amelioration of symptoms or a prolongation of survival in a subject. Toxicity and therapeutic efficacy of such molecules can be determined by standard pharmaceutical procedures in cell cultures or experimental animals, e.g., by determining the LD50 (the dose lethal to 50% of the population) and the ED50 (the dose therapeutically effective in 50% of the population). The dose ratio of toxic to therapeutic effects is the therapeutic index, which can be expressed as the ratio LD50/ ED50. Agents that exhibit high therapeutic indices are preferred.

The effective amount or therapeutically effective amount is the amount of the agent or pharmaceutical composition that will elicit the biological or medical response of a tissue, system, animal, or human that is being sought by the researcher, veterinarian, medical doctor, or other clinician, e.g., improved vascular function, improved cardiac function, etc.

Dosages preferably fall within a range of circulating concentrations that includes the ED50 with little or no toxicity. Dosages may vary within this range depending upon the dosage form employed and/or the route of administration utilized. The exact formulation, route of administration, dosage, and dosage interval should be chosen according to methods known in the art, in view of the specifics of a subject's condition.

Dosage amount and interval may be adjusted individually to provide plasma levels of the active moiety that are sufficient to achieve the desired effects, e.g., improved vascular function, improved cardiac function, etc, i.e., minimal effective concentration (MEC). The MEC will vary for each compound but can be estimated from, for example, in vitro data and animal experiments. Dosages necessary to achieve the MEC will depend on individual characteristics and route of administration. In cases of local administration or selective uptake, the effective local concentration of the drug may not be related to plasma concentration.

The amount of agent or composition administered may be dependent on a variety of factors, including the sex, age, and weight of the subject being treated, the severity of the affliction, the manner of administration, and the judgment of the prescribing physician.

The present compositions may, if desired, be presented in a pack or dispenser device containing one or more unit dosage forms containing the active ingredient. Such a pack or device may, for example, comprise metal or plastic foil, such as a blister pack, or glass and rubber stoppers such as in vials. The pack or dispenser device may be accompanied by instructions for administration. Compositions comprising a compound of the invention formulated in a compatible pharmaceutical carrier may also be prepared, placed in an appropriate container, and labeled for treatment of an indicated condition.

These and other embodiments of the present invention will readily occur to those of ordinary skill in the art in view of the disclosure herein.

### EXAMPLES

The invention will be further understood by reference to the following examples, which are intended to be purely exemplary of the invention. These examples are provided solely to illustrate the claimed invention. The present invention is not limited in scope by the exemplified embodiments, which are intended as illustrations of single aspects of the invention only. Any methods that are functionally equivalent are within the scope of the invention. Various modifications of the invention in addition to those described herein will become apparent to those skilled in the art from the foregoing description and accompanying figures.

### Example 1: Anti-CTGF Therapy Reduces Blood Pressure in Humans

Plasma levels of CTGF have been correlated to systolic blood pressure (Jaffa et al. (2005) American Society of Nephrology Annual Meeting Abstract, Journal of the American Society of Nephrology, November 2005). The effect of anti-CTGF antibody therapy on blood pressure was examined as follows. Human patients with type 1 diabetes and incipient nephropathy or with type 2 diabetes and incipient nephropathy were enrolled in an open-label Phase 1 study to examine the effects of anti-CTGF antibody therapy for treatment of diabetic nephropathy. Patients were previously taking various combinations of physician-prescribed blood pressure medications for a minimum of 1 month prior to administration of anti-CTGF antibody therapy of the current study. These blood pressure medications included angiotensin converting enzyme inhibitors (e.g., benazepril, fosinopril, lisinopril, quinapril), angiotensin II receptor blockers (e.g., losartan), beta-blockers (e.g., metoprolol tartrate, betaxolol, valsartan), diuretics (e.g., hydrochlorothiazide), and vasodilators (e.g., isosorbide dinitrate). Patients were maintained on these previously-administered blood pressure medications during the course of the present study.

Patients were administered anti-CTGF antibody (3 mg/kg) by intravenous infusion once every two weeks (i.e., on days 0, 14, 28, 42) for a total of 4 infusions. Blood pressure of each patient was measured at the beginning of the study (day 0) prior to administration of the first anti-CTGF antibody infusion, and again two weeks following the final administration of anti-CTGF antibody (day 56). No serious adverse events relating to anti-CTGF antibody administration were observed in any subject during the course of this study.

As shown in Tables 1, 2, and 3 below, subjects administered anti-CTGF antibody showed a reduction in blood pressure at day 56. Specifically, administration of anti-CTGF antibody reduced systolic blood pressure (Table 1), diastolic blood pressure (Table 2), and mean arterial pressure (Table 3) in subjects with type 1 diabetes with incipient nephropathy or type 2 diabetes with incipient nephropathy.

The average systolic blood pressure and diastolic blood pressure at day 0 was 133 mm Hg and 71 mm Hg, respectively. Two weeks following the final administration of anti-CTGF antibody, the average systolic blood pressure was 120 mm Hg and the average diastolic blood pressure was 67 mm Hg. These results showed that human subjects administered anti-CTGF antibody had an average reduction in systolic blood pressure of 13 mm Hg (a 9.7% reduction in systolic blood pressure) and an average reduction in diastolic blood pressure of 4 mm Hg (a 5.5% reduction in diastolic blood pressure).

Mean arterial pressure (MAP) was also reduced in human subjects administered anti-CTGF antibody. Specifically, a reduction in MAP from 113 mm Hg to 101 mm Hg (corresponding to a 10.6% reduction) was observed following the 56-day anti-CTGF antibody administration protocol described herein.

A reduction in pulse pressure (systolic blood pressure minus diastolic blood pressure) was also observed in human subjects treated with anti-CTGF antibody. (Data not shown.)

**TABLE 1**

| Patient Number | Systolic, Day 0 (mm Hg) | Systolic, Day 56 (mm Hg) | Change in Systolic Blood Pressure |
|---|---|---|---|
| 1 | 138 | 128 | -10 |
| 2 | 136 | 110 | -26 |
| 3 | 128 | 126 | -2 |
| 4 | 128 | 120 | -8 |
| 5 | 134 | 118 | -16 |
| 6 | 137 | 117 | -20 |
| 7 | 145 | 125 | -20 |
| 8 | 120 | 114 | -6 |

**TABLE2**

| Patient Number | Diastolic, Day 0 (mm Hg) | Diastolic, Day 56 (mm Hg) | Change in Diastolic Blood Pressure |
|---|---|---|---|
| 1 | 80 | 78 | -2 |
| 2 | 70 | 62 | -8 |
| 3 | 78 | 74 | -4 |
| 4 | 78 | 68 | -10 |
| 5 | 72 | 72 | 0 |
| 6 | 63 | 58 | -5 |
| 7 | 66 | 58 | -8 |
| 8 | 63 | 62 | -1 |

**TABLE 3**

| Patient Number | MAP, Day 0 | MAP, Day 56 | Change in MAP |
|---|---|---|---|
| 1 | 119 | 111 | -8 |
| 2 | 114 | 94 | -20 |
| 3 | 111 | 109 | -2 |
| 4 | 111 | 103 | -8 |
| 5 | 113 | 103 | -10 |
| 6 | 112 | 97 | -15 |
| 7 | 119 | 103 | -16 |
| 8 | 101 | 97 | -4 |

These results showed that anti-CTGF therapy was effective in reducing blood pressure in human subjects. Specifically, these results demonstrate that administration of an anti-CTGF antibody reduced both systolic and diastolic blood pressure in human subjects. Additionally, results of the present study indicated that methods and compounds of the present invention are useful for reducing blood pressure and treating hypertension associated with type 1 diabetes or type 2 diabetes, as well as for reducing blood pressure or hypertension associated with kidney disease.

### Example 2: Anti-CTGF Therapy Reduces Blood Pressure in an Animal Model of Diabetes

The methods and agents of the invention reduced blood pressure in an animal model of diabetes as described below. Diabetes mellitus was induced in Sprague Dawley rats by a single i.v. dose of 0.1 M citrate-buffered (pH 4.1) streptozotocin (STZ) (65 mg/kg) on day zero. Successful induction of diabetes in animals treated with STZ was confirmed on day 2 by an elevation in fasted blood glucose levels (>250mg/dl).

Diabetes and disorders associated with diabetes were allowed to progress in the animals for 6 weeks following the STZ injection. After 6 weeks, diabetic animals were then divided into various treatment groups as follows: control human IgG (10 mg/kg, intra-peritoneal (IP) injection, three times per week for 6 weeks); anti-CTGF antibody (CLN-1, 10 mg/kg, IP injection, three times per week for 6 weeks); Captopril (75 *mg*/*kg*/*day, per os* (PO, oral administration), in drinking water); Losartan (20 mg/kg/day, PO).

Six weeks after treatment initiation, blood pressure was measured in non-anesthetized animals using the CODA System (Kent Scientific), which automatically performs rapid multiple measurements of six (6) hemodynamic parameters, including systolic pressure, diastolic pressure, mean pressure, heart pulse rate, blood volume, and blood flow. Animals were placed in a restrainer and allowed to acclimatize for 10 minutes. Ten separate measurements were obtained from each animal, and the average value for the last five measurements was used for subsequent analysis.

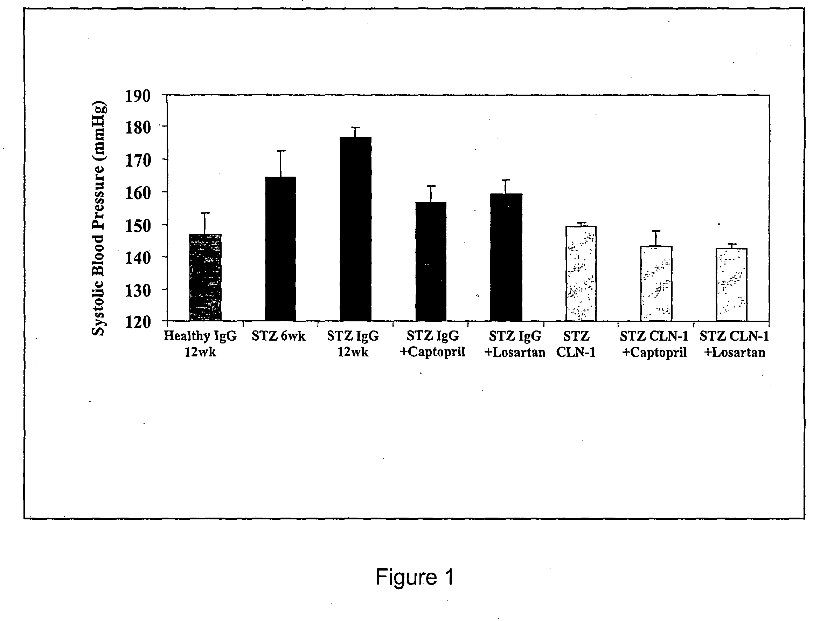

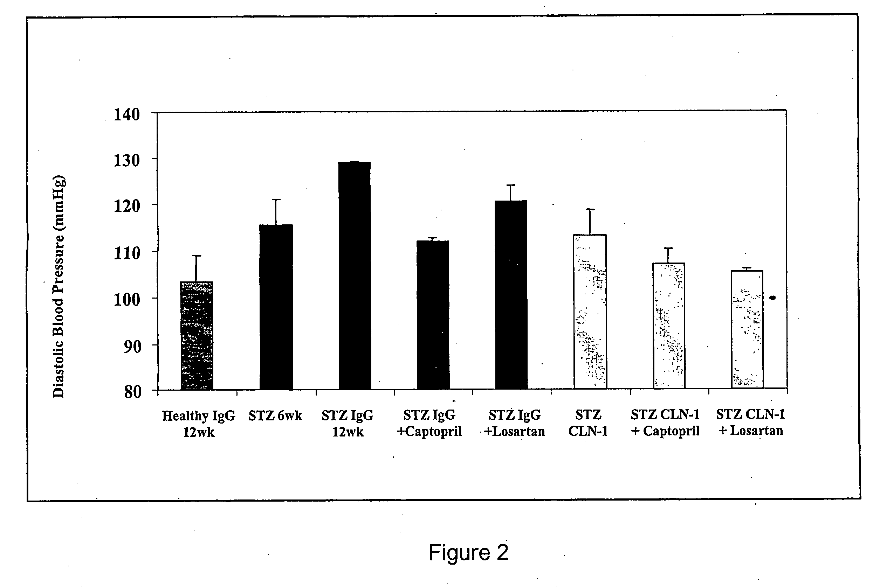

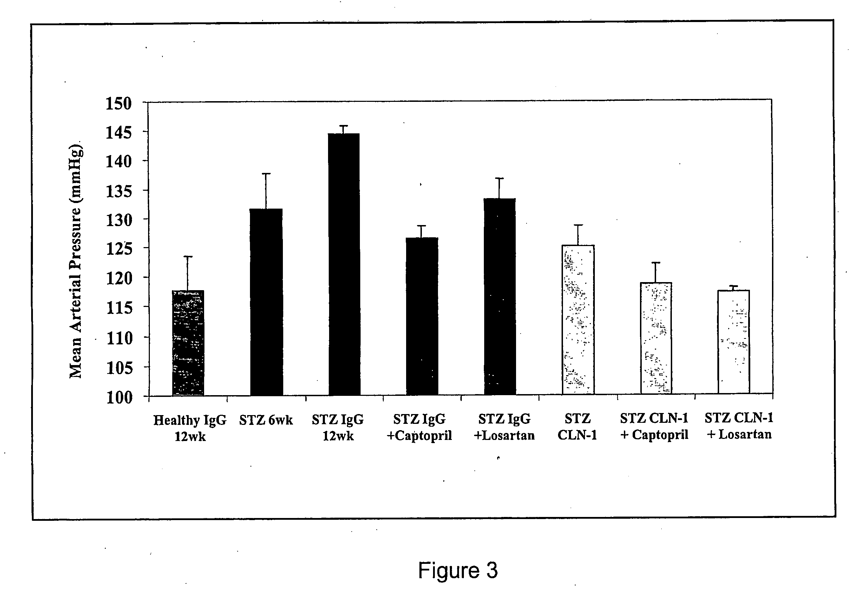

As shown in Figures 1, 2, and 3, respectively, systolic blood pressure, diastolic blood pressure, and mean arterial pressure (MAP) were elevated in diabetic animals compared to that of non-diabetic control animals at 6 and 12 weeks of diabetes. In contrast the values for blood pressure measurements observed in animals treated with anti-CTGF antibody were comparable to that observed in healthy control animals.

These results showed that treatment of diabetic animals with anti-CTGF antibody prevented and reversed pathologic increases in blood pressure. Further, treatment with anti-CTGF antibody was effective at reversing hypertension associated with diabetes. Therefore, inhibition of CTGF provides an effective therapeutic approach for reducing blood pressure and for treating hypertension.

### Example 3: Anti-CTGF Therapy Reduced Angiotensin II type 1 Receptor Gene Expression mRNA analysis

The effect of anti-CTGF therapy on expression of genes associated with blood pressure regulation was performed as follows. The animal model of diabetes described above in Example 2 was used. A portion of the heart and carotid artery was removed from each animal and placed in RNAlater for subsequent isolation and analysis of mRNA. RNA isolation and cDNA synthesis was carried out using the following protocol. Carotid artery tissue was added to TRIzol (Invitrogen) and homogenized with a 5 mm stainless steel bead for 8 min at 25 Htz in a Mixer Mill 300 (Qiagen). The homogenates were extracted with chloroform according to the manufacturer's instructions and aqueous supernatants were isolated. The aqueous supernatants were combined with equal volumes of 70% ethanol and then loaded on to RNeasy Mini spin columns (Qiagen). RNA was isolated according to the manufacturer's instructions. A similar protocol was followed for heart tissue with the exception that only a section from the left ventricular free wall region of the heart was used for RNA isolation. Synthesis of cDNA from total RNA was done using the Omniscript reverse transcriptase (Qiagen) and random primers according to the manufacturer's instructions.

Analysis of gene expression levels for various genes associated with blood pressure regulation was performed by quantitative PCR using TaqMan Universal PCR Master Mix (Applied Biosystems) and TaqMan Assay-on-Demand assays (Applied Biosystems) in a Prism 7000 system instrument (Applied Biosystems), according to manufacturer's instructions.

Examination of gene expression of the following genes was performed: angiotensin II receptor type 1 (Rn02132799_s1) and 18S ribosomal RNA (Hs99999901_s1).

Each PCR run included a standard curve and water blank. Gene expression data for angiotensin II type I receptor was normalized relative to the expression level of 18S ribosomal RNA for that sample.

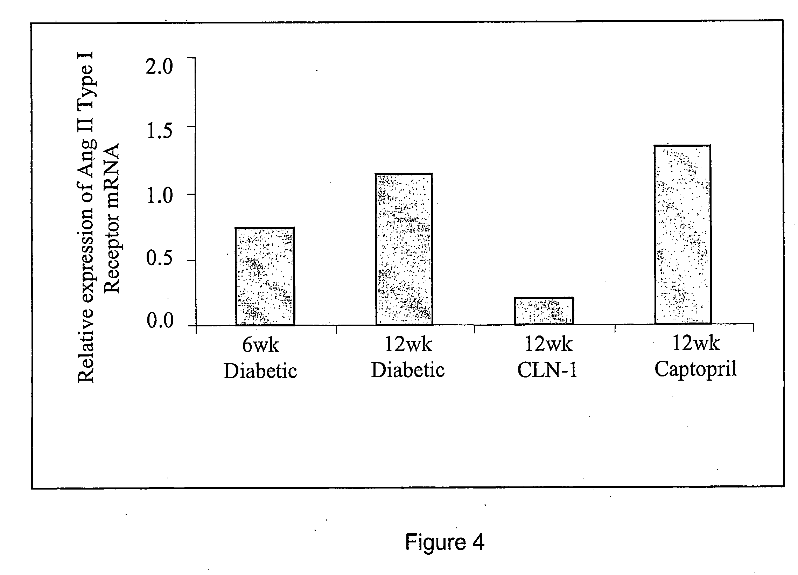

As shown in Figure 4, at 6 and 12 weeks following induction of diabetes, non-treated diabetic animals had elevated mRNA levels of angiotensin II type 1 receptor in the carotid artery. Animals administered anti-CTGF antibody from week 6 to week 12 showed reduced levels of angiotensin II type 1 receptor mRNA in the carotid artery compared to non-treated control animals. This data showed that anti-CTGF therapy is effective at reducing gene expression of angiotensin II type 1 receptor in arteries. This data indicated that anti-CTGF therapy is effective at reducing expression of genes associated with increased blood pressure.

Various modifications of the invention, in addition to those shown and described herein, will become apparent to those skilled in the art from the foregoing description.

## Claims

1. An agent that inhibits connective tissue growth factor (CTGF) for use in reducing blood pressure in a subject having elevated blood pressure, high blood pressure or hypertension, wherein the agent is an antibody that binds to CTGF, an antisense molecule that inhibits CTGF expression or an siRNA that inhibits CTGF expression.

2. The agent of claim 1 for the use of that claim, wherein the subject is a human subject.

3. The agent of claim 2 for the use of that claim, wherein the subject has or is at risk for having hypertension or diabetes.

4. The agent of claim 2 for the use of that claim, wherein the subject is a subject previously treated with or currently taking one or more blood pressure medications.

5. The agent according to any one of the preceding claims for the use of that claim, wherein the agent is for treating or preventing a disorder associated with elevated blood pressure or hypertension in the subject.

6. The agent according to any one of the preceding claims for the use of that claim, wherein the subject has a kidney disease or a nephropathy.

7. The agent of claim 6 for the use of that claim, wherein the subject has diabetic nephropathy.

8. The agent according to any one of the preceding claims for the use of that claim, wherein the agent is a human monoclonal antibody directed against CTGF.

9. The agent according to claim 8 for the use of that claim, wherein the human monoclonal antibody is CLN-1.

10. The agent according to any one of the preceding claims for the use of that claim, wherein the agent is to be used in combination with another therapeutic approach to treat elevated or high blood pressure, hypertension, or associated disorders and complications.

11. The agent according to claim 10 for the use of that claim, wherein the therapeutic approach is administration of a therapeutic agent selected from the group consisting of angiotensin converting enzyme inhibitors, angiotensin II receptor blockers, beta-blockers, alpha-blockers, calcium channel blockers, diuretics, and vasodilators.

12. The agent according to one any of the preceding claims for the use of that claim, wherein the agent is for reducing expression of a gene associated with increased blood pressure in the subject.

13. The agent according to claim 12 for the use of that claim, wherein the gene is angiotensin II type I receptor.

14. Use of an agent that inhibits connective tissue growth factor (CTGF), in the manufacture of a medicament for reducing blood pressure in a subject having elevated blood pressure, high blood pressure or hypertension, wherein the agent is an antibody that binds to CTGF, an antisense molecule that inhibits CTGF expression or an siRNA that inhibits CTGF expression.

## Patentansprüche

1. Mittel, das Bindegewebewachstumsfaktor (CTGF, engl. connective tissue growth factor) hemmt, für die Verwendung zur Verringerung des Blutdrucks in einem Individuum mit erhöhtem Blutdruck, hohem Blutdruck oder Hypertonie, wobei das Mittel ein Antikörper, der an CTGF bindet, ein Antisense-Molekül, das die CTGF-Expression hemmt, oder eine siRNA, die die CTGF-Expression hemmt, ist.

2. Mittel nach Anspruch 1 für die Verwendung nach diesem Anspruch, wobei das Individuum ein menschliches Individuum ist.

3. Mittel nach Anspruch 2 für die Verwendung nach diesem Anspruch, wobei das Individuum Hypertonie oder Diabetes hat oder einem Risiko ausgesetzt ist, Hypertonie oder Diabetes zu bekommen.

4. Mittel nach Anspruch 2 für die Verwendung nach diesem Anspruch, wobei das Individuum eines ist, das zuvor mit einer oder mehreren Blutdruckmedikationen behandelt wurde oder diese zurzeit einnimmt.

5. Mittel nach einem der vorhergehenden Ansprüche für die Verwendung nach diesem Anspruch, wobei das Mittel zur Behandlung oder Vorbeugung einer Störung dient, die mit erhöhtem Blutdruck oder Hypertonie in dem Individuum einhergeht.

6. Mittel nach einem der vorhergehenden Ansprüche für die Verwendung nach diesem Anspruch, wobei das Individuum eine Nierenkrankheit oder eine Nephropathie hat.

7. Mittel nach Anspruch 6 für die Verwendung nach diesem Anspruch, wobei das Individuum diabetische Nephropathie hat.

8. Mittel nach einem der vorhergehenden Ansprüche für die Verwendung nach diesem Anspruch, wobei das Mittel ein gegen CTGF gerichteter humaner monoklonaler Antikörper ist.

9. Mittel nach Anspruch 8 für die Verwendung nach diesem Anspruch, wobei es sich bei dem humanen monoklonalen Antikörper um CLN-1 handelt.

10. Mittel nach einem der vorhergehenden Ansprüche für die Verwendung nach diesem Anspruch, wobei das Mittel in Kombination mit einem anderen Therapieansatz zur Behandlung eines erhöhten oder hohen Blutdrucks, von Hypertonie oder damit einhergehenden Störungen oder Komplikationen zu verwenden ist.

11. Mittel nach Anspruch 10 für die Verwendung nach diesem Anspruch, wobei der Therapieansatz die Verabreichung eines Therapeutikums ist, das aus der Gruppe ausgewählt wird, die aus Inhibitoren des Angiotensin-konvertierenden Enzyms, Angiotensin-II-Rezeptor-Blockern, Betablockern, Alphablockern, Calciumkanal-Blockern, Diuretika und Vasodilatatoren besteht.

12. Mittel nach einem der vorhergehenden Ansprüche für die Verwendung nach diesem Anspruch, wobei das Mittel zur Verringerung der Expression eines Gens, das mit erhöhtem Blutdruck in dem Individuum in Zusammenhang steht, dient.

13. Mittel nach Anspruch 12 für die Verwendung nach diesem Anspruch, wobei es sich um das Gen des Angiotensin II Typ I-Rezeptors handelt.

14. Verwendung eines Mittels, das Bindegewebewachstumsfaktor (CTGF) hemmt, für die Herstellung eines Arzneimittels zur Verringerung des Blutdrucks in einem Individuum mit erhöhtem Blutdruck, hohem Blutdruck oder Hypertonie, wobei das Mittel ein Antikörper, der an CTGF bindet, ein Antisense-Molekül, das die CTGF-Expression hemmt, oder eine siRNA ist, die die CTGF-Expression hemmt.

## Revendications

1. Agent qui inhibe le facteur de croissance du tissu conjonctif (CTGF) pour utilisation dans la réduction de la pression artérielle chez un sujet ayant une pression artérielle augmentée, une pression artérielle élevée ou une hypertension, où l'agent est un anticorps qui se lie à CTGF, une molécule antisens qui inhibe l'expression de CTGF ou un ARNsi qui inhibe l'expression de CTGF.

2. Agent de la revendication 1 pour l'utilisation de cette revendication, où le sujet est un sujet humain.

3. Agent de la revendication 2 pour l'utilisation de cette revendication, où le sujet a ou est à risque d'avoir de l'hypertension ou du diabète.

4. Agent de la revendication 2 pour l'utilisation de cette revendication, dans lequel le sujet est un sujet précédemment traité avec ou prenant actuellement un ou plusieurs médicaments régulant la pression artérielle.

5. Agent selon l'une quelconque des revendications précédentes pour l'utilisation de cette revendication, où l'agent est destiné à traiter ou prévenir un trouble associé à une pression artérielle augmentée ou une hypertension chez le sujet.

6. Agent selon l'une quelconque des revendications précédentes pour l'utilisation de cette revendication, où le sujet a une maladie rénale ou une néphropathie.

7. Agent de la revendication 6 pour l'utilisation de cette revendication, où le sujet a une néphropathie diabétique.

8. Agent selon l'une quelconque des revendications précédentes pour l'utilisation de cette revendication, où l'agent est un anticorps monoclonal humain dirigé contre CTGF.

9. Agent selon la revendication 8 pour l'utilisation de cette revendication, où l'anticorps monoclonal humain est CLN-1.

10. Agent selon l'une quelconque des revendications précédentes pour l'utilisation de cette revendication, où l'agent doit être utilisé en combinaison avec une autre approche thérapeutique pour traiter une pression artérielle augmentée ou élevée, une hypertension, ou des troubles ou complications associés.

11. Agent selon la revendication 10 pour l'utilisation de cette revendication, où l'approche thérapeutique est l'administration d'un agent thérapeutique choisi dans le groupe constitué d'inhibiteurs d'enzyme de conversion d'angiotensine, d'inhibiteurs de récepteur d'angiotensine II, de bêtabloquants, d'alpha-bloquants, d'inhibiteurs calciques, de diurétiques et de vasodilatateurs.

12. Agent selon l'une quelconque des revendications précédentes pour l'utilisation de cette revendication, où l'agent est destiné à réduire l'expression d'un gène associé à une pression artérielle augmentée chez le sujet.

13. Agent selon la revendication 12 pour l'utilisation de cette revendication, où le gène est le récepteur de type I d'angiotensine II.

14. Utilisation d'un agent qui inhibe le facteur de croissance du tissu conjonctif (CTGF) dans la fabrication d'un médicament pour produire la pression artérielle chez un sujet ayant une pression artérielle augmentée, une pression artérielle élevée ou une hypertension, où l'agent est un anticorps qui se lie à CTGF, une molécule antisens qui inhibe l'expression de CTGF ou un ARNsi qui inhibe l'expression de CTGF.
